Europäisches Patentamt

European Patent Office

Office européen des brevets

(19)

(11) Veröffentlichungsnummer: **0 230 265**
**A2**

## (12) EUROPÄISCHE PATENTANMELDUNG

(21) Anmeldenummer: **87100420.6**

(22) Anmeldetag: **14.01.87**

(51) Int. Cl.⁴: **A 61 K 39/12**
**A 61 K 47/00**

(30) Priorität: **23.01.86 DE 3601922**

(43) Veröffentlichungstag der Anmeldung:
**29.07.87 Patentblatt 87/31**

(84) Benannte Vertragsstaaten:
**AT BE CH DE FR GB IT LI LU NL SE**

(71) Anmelder: **BEHRINGWERKE AKTIENGESELLSCHAFT**
**Postfach 1140**
**D-3550 Marburg/Lahn (DE)**

(72) Erfinder: **Bernhardt, Dieter, Dr.**
**Goldbergstrasse 18a**
**D-3553 Cölbe (DE)**

(74) Vertreter: **Meyer-Dulheuer, Karl-Hermann, Dr. et al**
**HOECHST Aktiengesellschaft Zentrale Patentabteilung**
**Postfach 80 03 20**
**D-6230 Frankfurt/Main 80 (DE)**

(54) **Stabilisiertes Virus-Antigen, Verfahren zu seiner Herstellung und seine Verwendung.**

(57) Es wird ein Verfahren zur Stabilisierung von Virus-Antigenen beschrieben, wobei die Virussuspension mit einer abgebauten und vernetzten Gelatine, einem Disaccharid und einem Pepton versetzt und gegebenenfalls zur Trockene gebracht wird. Auf diese Weise hergestellte Zubereitungen können in einer Vaccine oder einem Diagnostikum verwendet werden.

EP 0 230 265 A2

Bundesdruckerei Berlin

## Beschreibung

Stabilisiertes Virus-Antigen, Verfahren zu seiner Herstellung und seine Verwendung

Die Erfindung betrifft Zubereitungen von Virus-Antigenen, die zur Stabilisierung des Antigens gegen einen Aktivitätsverlust ein Gelatinederivat, ein Disaccarid und ein Caseinhydrolysatpepton enthalten, sowie ein Verfahren zu ihrer Herstellung. Solche Zubereitungen von Virus-Antigenen können in Vaccinen oder Diagnostika verwendet werden.

Verfahren zur Stabilisierung von Virus-Antigenen sind bekannt. So ist in der US-Patentschrift 4,337,242 beschrieben, daß Virus-Vaccinen durch Zusatz hydrolysierter Gelatine mit einem Molekulargewicht von etwa 3.000, eines Mono- oder Disaccarids, eines Zellkulturmediums, von L-Glutaminsäure und L-Arginin in einer Pufferlösung stabilisiert werden können. Die in dieser Schrift angegebenen Ergebnisse von Stabilisierungsversuchen lassen erkennen, daß die Stabilisierung von flüssigen Vaccinen, die auf die beschriebene Weise stabilisiert wurden, unter Temperaturbelastung unzureichend ist.

Es sind auch bereits eine abgebaute und vernetzte Gelatine (DE-AS 11 83 629), Lactose (EP 0 065 905) oder Natriumglutaminat (DE-AS 11 83 629) zur Stabilisierung von Virus-Antigenen verwendet worden. Doch war auch in diesen Fällen das Ergebnis unbefriedigend.

Es bestand besonders das Bedürfnis, Masern-, Mumps-, Röteln-, VD/MD(Mucosal disease/Virusdiarrhoe)-, PI3 (Parainfluenza Typ 3)- und IBR/IPV(Infectiöse Bovine Rhinotracheitis/Infectiöse Pustulöse Vulvovaginitis)Virus sowie BRSV (Bovine Respiratory Syncytial Virus) besser zu stabilisieren.

Es wurde nun überraschenderweise gefunden, daß Virus-Antigene gegen Aktivitätsverlust während einer Gefriertrocknung und Temperaturbelastung geschützt werden können, indem eine abgebaute und vernetzte Gelatine, ein Disaccharid und ein Caseinhydrolysatpepton zugesetzt werden.

Gegenstand der Erfindung ist deshalb eine Zubereitung eines Virusantigens, dadurch gekennzeichnet, daß sie mindestens das Antigen, eine abgebaute und vernetzte Gelatine, ein Disaccharid und ein Caseinhydrolysatpepton enthält.

Diese Zubereitung kann flüssig oder fest sein.

Das Disaccharid ist vorzugsweise Saccharose.

Gegenstand der Erfindung ist auch ein Verfahren zur Stabilisierung eines Virus-Antigens, dadurch gekennzeichnet, daß einer Suspension des Virus eine abgebaute und vernetzte Gelatine, ein Disaccharid und Caseinhydrolysatpepton zugesetzt und gegebenenfalls die Suspension zur Trockene gebracht, vorzugsweise gefriergetrocknet wird.

Ein auf diese Weise stabilisiertes Virus ergibt eine höhere Virusantigenausbeute in einem Gefrier-Tau-Zyklus. Die Antigenverluste bei einer Zentrifugation oder Filtration werden verringert. Weiterhin treten geringere Verluste während einer Gefriertrocknung einer Antigensuspension und bei einer Temperaturbelastung des getrockneten Antigens auf.

Als abgebaute und vernetzte Gelatine wird vorzugsweise eine solche gebraucht, die durch Abbau von Kollagen oder Gelatine bis zu einem Molekulargewicht von 2.000 bis 35.000 und chemische Vernetzung, vorzugsweise mit einem Diisocyanat, entsteht. Es können auch Kollagenabbaupro dukte oder Gelatine zunächst vernetzt und dann weiter bis zu einem Molekulargewicht von 10.000 bis 100.000 abgebaut werden.

Besonders geeignete Kollagenprodukte sind die aus DE-P 11 18 792 und DE-P 11 55 134 bekannten. Sie werden hergestellt, indem man entweder Kollagen oder dessen Abbauprodukte in wässriger Lösung bei einer Temperatur von 60 bis 150°C bis zu einem Molekulargewicht von 2.000 bis 20.000, vorzugsweise 5.000 bis 10.000, abbaut, das abgebaute Kollagen mit einem Diisocyanat bei einer Temperatur von 0 bis 100°C im neutralen bis schwach alkalischen Medium, gegebenenfalls in Gegenwart inerter organischer Lösungsmittel in der Weise umgesetzt, daß die angewandte Isocyanatmenge geringer ist als die stöchiometrische, welche sich aus der Anzahl der im abgebauten Kollagen vorhandenen Amino- und Guanidinogruppen errechnet, und vorzugsweise etwa 20 bis 80% dieser Menge beträgt, und dann das entstandene Vernetzungsprodukt auf einen pH-Wert von etwa 7 einstellt; oder daß man Kollagenabbauprodukte mit einem Diisocyanat bei einer Temperatur von 0 bis 100°C im neutralen bis schwach alkalischen Medium, gegebenenfalls in Gegenwart inerter organischer Lösungsmittel umsetzt, wobei die angewandte Isocyanatmenge 20 bis 80% der stöchiometrischen beträgt, die sich aus der Anzahl der vorhandenen Amino- und Guanidinogruppen errechnet, die entstandenen Vernetzungsprodukte in wässriger Lösung bei einer Temperatur von 60 bis 150°C bis zu einem Molekulargewicht von 10.000 bis 100.000 abbaut und die erhaltenen Lösungen auf einen pH-Wert von 7 einstellt. Als 3,5%ige Lösung bei einem mittleren Molekulargewicht von 35.000 sind diese auch als Infusionslösung zur Plasmasubstitution im Handel ("Polygeline").

Die vernetzte Gelatine wird in einer Menge zugegeben, daß 5-100 g, vorzugsweise 20-50 g, in einem Liter Virus-Antigen enthaltender Flüssigkeit vorhanden sind. Das Disaccharid wird in einer Menge von 20-300 g, vorzugsweise 100-200 g, in einem Liter Flüssigkeit verwendet. Das Caseinhydrolysatpepton wird in einer Menge von 20-400 g, vorzugsweise 100-200 g, in einem Liter Flüssigkeit verwendet.

Gegebenenfalls kann das Flüssigkeitsgemisch zur Trockene gebracht, vorzugsweise gefriergetrocknet, werden.

Als Caseinhydrolysatpepton wird vorzugsweise pankreatisch verdautes Casein, besonders CHP 140 der Fa. Gibco gebraucht.

**Beispiel 1**

Je 20 ml einer Suspension von MD/VD Virus (Mucosal disease/Virusdiarrhoe), Stamm Ug 59 wurden mit 3 ml einer wässrigen Lösung, die 500 g/l CHP 140 (Caseinhydrolysatpepton der Firma Gibco) enthielt (Lösung A), mit 3 ml einer wässrigen Lösung, die 50 g/l abgebaute und vernetzte Gelatine (DE-PS 11 18 792 oder 11 55 134) und 500 g/l Saccharose enthielt (Lösung B), oder mit 3 ml einer Lösung, die in einem Liter 964 ml Eagle Medium 1959 35 ml einer sterilen Lösung von 5 g Natriumkarbonat in 100 ml Wasser und 1 ml einer Lösung von 12.5 mg Gentamycinsulfat in 100 ml Wasser enthielt (EM; Lösung C), oder mit je 3 ml der Lösungen A, B und C versetzt. Je 2 ml wurden lyophilisiert, thermobelastet (56°) und danach der Gehalt an infektiösem Virus bestimmt.

Die Ergebnisse zeigen die folgenden Tabellen (die Zahlen bedeuten Virustiter in log 10/ml):

## Tabelle 1

|  | CHP 140 | Saccharose + Gelatine | Saccharose + Gelatine + CHP 140 |
|---|---|---|---|
|  | Lösung A | Lösung B | Lösung A + B |
| Vor Lyophilisation | 6,7 | 6,7 | 6,7 |
| nach Lyophilisation | 5,3 | 6,5 | 6,3 |
| 2 Std. 56°C | 5,5 | 4,7 | 5,3 |
| 4 Std. 56°C | 5,1 | 4,7 | 5,1 |
| 7 Std. 56°C | 4,5 | 3,9 | 4,7 |
| 16 Std. 56°C | 2,5 | 0,0 | 4,1 |

Die Tabelle zeigt, daß durch die Kombination von CHP 140, Saccharose und Gelatine eine nicht zu erwartende Steigerung der Thermostabilisierung bewirkt wird.

**Beispiel 2**

Je 20 ml MD/VD-Virus-Suspension in Eagles-Medium 1959 wurden mit 6,3 oder 1,5 ml einer wässrigen Lösung, die 250 g/l CHP 140, 25 g/l Polygeline und 125 g/l Saccharose enthielt ("Stabilisatorlösung") versetzt. Je 2 ml wurden lyophilisiert, thermobehandelt (56°C) und titriert.

## Tabelle 2

|  | Stabilisatormenge (ml) | | | |
|---|---|---|---|---|
|  | 6 | 3 | 1.5 | 0 |
| Vor Lyophilisation | 4,7 | 4,7 | 4,7 | 4,7 |
| nach Lyophilisation | 4,5 | 4,5 | 4,9 | 2,7 |
| 2 Std. 56°C | 4,1 | 3,7 | 3,5 | 0 |
| 4 Std. 56°C | 4,1 | 3,7 | 3,7 | 0 |
| 7 Std. 56°C | 3,9 | 3,3 | 2,9 | 0 |
| 16 Std. 56°C | 2,9 | 2,5 | 2,3 | 0 |

Die Werte sind Durchschnittswerte aus 5 Bestimmungen. Wie aus diesem Beispiel hervorgeht, entfaltet die Stabilisatorlösung über einen weiten Konzentrationsbereich ihre Wirkung.

Beispiel 3

Je 50 ml Masernvirus-Suspension wurden mit 15 ml Stabilisatorlösung oder 15 ml Mediumgemisch EM versetzt. Danach wurden beide Proben bei -80°C eingefroren. Nach dem Autauen wurde der Virusgehalt festgestellt.

Titer der Virussuspension mit Stabilisator: $10^{7,1}$/ml

Titer der Virussuspension ohne Stabilisator: $10^{5,6}$/ml

Beispiel 4

Die beiden Proben von Beispiel 3 wurden zentrifugiert, um Zellfragmente aus der Virussuspension zu entfernen. Dann wurde der Überstand titriert.

Titer der Virussuspension mit Stabilisator: 7,0/ml $KID_{50}$.

Titer der Virussuspension ohne Stabilisator: 5,6/ml $KID_{50}$.

Beispiel 5

Der Überstand der wie in Beispiel 4 behandelten Proben wurde membranfiltriert (Satorius 0,45 µ) und das Filtrat titriert.

Titer der Virussuspension mit Stabilisator: $10^{6,7}$/ml $KID_{50}$.

Titer der Virussuspension ohne Stabilisator: $10^{5,0}$/ml $KID_{50}$.

Beispiel 6

Je 20 ml von Suspensionen verschiedener Viren wurden mit 6 ml Stabilisatorlösung aus Beispiel 2 oder mit 6 ml Saccharose/ Gelatinelösung (Beispiel 1), versetzt.

Die Proben wurden zu 2 ml abgefüllt, lyophilisiert, thermobelastet (56°C) und titriert. Das Ergebnis ist in Tabelle 3 gezeigt.

Tabelle 3

| | vor Lyophilisation | nach 56°C | 2 Std. 56°C | 4 Std. 56°C | 7 Std. 56°C | 16 Std. 56°C | Zu-satz* |
|---|---|---|---|---|---|---|---|
| Masern V | 5,7 | 5,7 | 5,3 | 4,3 | 3,9 | 2,5 | 1 |
| Masern V | 5,7 | 5,3 | 3,7 | 2,7 | 2,5 | 0,0 | 2 |
| Mumps V | 4,5 | 3,7 | 3,5 | 3,3 | 2,3 | 0,0 | 1 |
| Mumps V | 4,5 | 3,3 | 2,5 | 0,0 | 0,0 | 0,0 | 2 |
| RSV | 3,5 | 3,1 | 3,1 | 2,7 | 2,7 | 2,5 | 1 |
| RSV | 3,5 | 3,7 | 3,3 | 3,1 | 2,5 | 0,0 | 2 |
| IBR | 6,9 | 6,7 | 6,3 | 5,7 | 5,7 | 5,9 | 1 |
| IBR | 6,9 | 7,1 | 5,9 | 5,9 | 5,9 | 4,5 | 2 |
| PI$_3$ | 6,7 | 6,5 | 5,1 | 4,7 | 4,1 | 2,9 | 1 |
| PI$_3$ | 6,7 | 6.3 | 5,5 | 5,1 | 4,3 | 0,0 | 2 |
| Röteln V | 4,5 | 4,7 | 4,9 | 4,7 | 4,9 | 4,7 | 1 |
| Röteln V | 4,5 | 4,1 | 3,3 | 0,0 | 0,0 | 0,0 | 2 |
| VD/MD | 4,9 | 4,7 | 4,3 | 3,9 | 3,5 | 3,3 | 1 |
| VD/MD | 4,9 | 4,7 | 3,7 | 3,7 | 3,3 | 0,0 | 2 |

\* 1 = Stabilisatorlösung (Beispiel 2)  
  2 = Saccharose/Gelatinelösung (Lösung B)

0 230 265

**Patentansprüche**

1. Zubereitung eines Virus-Antigens, dadurch gekennzeichnet, daß sie mindestens das Antigen, eine abgebaute und vernetzte Gelatine, ein Disaccharid und ein Caseinpepton enthält.

2. Zubereitung nach Anspruch 1, dadurch gekennzeichnet, daß sie 5-100 g, vorzugsweise 20-50 g, abgebaute und vernetzte Gelatine, 20-300 g, vorzugsweise 100-200 g Disaccharid und 20-400 g, vorzugsweise 100-200 g Caseinhydrolysatpepton in einem Liter Virus-Antigen enthaltender Flüssigkeit enthält.

3. Zubereitung nach Anspruch 1, dadurch gekennzeichnet, daß sie gefriergetrocknet ist.

4. Zubereitung nach Anspruch 1, dadurch gekennzeichnet, daß die abgebaute und vernetzte Gelatine eine solche ist, die durch Abbau von Kollagen bis zu einem Molekulargewicht von 2.000 bis 35.000 und chemische Vernetzung hergestellt wird.

5. Zubereitung nach Anspruch 1, dadurch gekennzeichnet, daß das Disaccharid Saccharose ist.

6. Zubereitung nach Anspruch 1, dadurch gekennzeichnet, daß das Virus-Antigen Masern-, Röteln- oder Mumpsvirus ist.

7. Verfahren zur Herstellung einer Zubereitung nach Anspruch 1, dadurch gekennzeichnet, daß einer Suspension eines Virus eine abgebaute und vernetzte Gelatine, ein Disaccharid und ein Caseinhydrolysatpepton zugesetzt und gegebenenfalls die Suspension zur Trockene gebracht, vorzugsweise gefriergetrocknet, wird.

8. Verwendung einer Zubereitung nach Anspruch 1 in einem Impfstoff oder Diagnostikum